# EUROPÄISCHE PATENTSCHRIFT

(11) **EP 2 200 497 B1**
(45) Veröffentlichungstag und Bekanntmachung des Hinweises auf die Patenterteilung: **05.03.2014**
(21) Anmeldenummer: 08758085.8
(22) Anmeldetag: 15.05.2008
(51) Int. Cl.: A61B 1/00, A61B 1/012, A61B 1/313, A61M 25/00, A61M 25/06, G02B 23/24

(54) **MODULARES ENDOSKOP**
MODULAR ENDOSCOPE
ENDOSCOPE MODULAIRE

(30) Priorität: 23.08.2007 DE 202007011781 U; 27.02.2008 DE 102008011387
(43) Veröffentlichungstag der Anmeldung: 30.06.2010
(73) Patentinhaber: POLYDIAGNOST ENTWICKLUNGS-, PRODUKTIONS-, VERTRIEBS-, UND SERVICE GESELLSCHAFT MBH, 85276 Pfaffenhofen (DE)
(72) Erfinder: SCHAAF, Hansgeorg, 85293 Reichertshausen (DE)
(74) Vertreter: Winter, Brandl, Fürniss, Hübner, Röss, Kaiser, Polte - Partnerschaft
(86) Internationale Anmeldenummer: PCT/DE2008/000843
(87) Internationale Veröffentlichungsnummer: WO 2009/024107

(56) Entgegenhaltungen:
- WO-A-2006/027136
- DE-A1-102004 055 412
- DE-A1-102006 022 827
- US-A- 5 735 792
- US-A- 6 007 531
- US-A1- 2004 064 021
- DATABASE MEDLINE [Online] US NATIONAL LIBRARY OF MEDICINE (NLM), BETHESDA, MD, US; März 1987 (1987-03), BAGLEY D H: "Flexible ureteropyeloscopy with modular, "disposable" endoscope." XP002497824 Database accession no. NLM3824726 & UROLOGY MAR 1987, Bd. 29, Nr. 3, März 1987 (1987-03), Seiten 296-300, ISSN: 0090-4295

## Beschreibung

Die Erfindung betrifft ein modulares Endoskop gemäß dem Oberbegriff des Anspruchs 1. Ein derartiges Endoskop ist aus der US 6007531 A bekannt.

Die Endoskopie hat sich im Rahmen der minimal invasiven Chirurgie fest etabliert. Dabei werden Endoskope für Diagnostik, Therapie und mit Kathetersonden ausgeführte Endoskope eingesetzt. In der Diagnostik und Therapie eingesetzte Endoskope haben eine Kanüle oder Sonde, in die eine Optik zur Licht- und Bildübertragung eingesetzt ist und die mit weiteren Anschlüssen, beispielsweise einem Spülanschluss zur Zuführung von Spülflüssigkeit oder einem Arbeitsanschluss zum Ansetzen eines Werkzeugs, beispielsweise eines Bohrers, einer Biopsiezange oder eines Laserstrahlleiters zur chirurgischen oder therapeutischen Behandlung ausgeführt ist. Diese bekannten Endoskope sind auf den jeweiligen Einsatzbereich hin, beispielsweise die Endoskopie kleiner Gelenke (Kiefer-, Fuß-, Zehen-, Fingergelenk), Wurzelkanal-Endoskopie, Milchkanal-Endoskopie, Zahntaschen-Endoskopie, Tränenkanal-Endoskopie, die Bandscheiben- oder Spinalkanal-Endoskopie optimiert, wobei der Außendurchmesser je nach Einsatzbereich zwischen 0.3 mm (Glaukom-Diagnostik) bis in den Bereich von mehreren Millimetern bei Kathetersonden liegen. Derartige Endoskope sind beispielsweise in der DE 20 2006006 322 U1, der DE 195 42 955 C2 oder der DE 10 2005 018 825 B3 offenbart, die sämtlich auf die Anmelderin zurückgehen. In der DE 10 2004 055412 A1 ist ein Endoskop beschrieben, bei dem ein Kathetersystem durch einen Schutzschlauch gebildet ist, in den ein Lichtleiter zur Laserbehandlung von Blutgefäßen eingeschoben wird.

In der DE 10 2006 022 827 A1 ist ein Endoskop beschrieben, bei dem in einer Kanüle eine Optik zur Bildübertragung, eine Beleuchtungsoptik und ein Spülkanal aufgenommen sind.

Ein großes Problem in der Medizintechnik, insbesondere auch bei der Endoskopie ist die Dekontamination der Instrumente. So werden derzeit bei Untersuchungen von Hepatitis-B-, AIDS- oder Kreuzfeld-Jakob-Patienten die Endoskope nach der Untersuchung vernichtet. Ansonsten ist eine sorgfältige Sterilisation der chirurgischen und diagnostischen Instrumente erforderlich, um Mikroorganismen oder sonstige Verunreinigungen auf der Oberfläche der Instrumente abzutöten bzw. zu beseitigen. Proble-matisch ist dabei insbesondere die Reinigung der Kupplungselemente bzw. der Arbeitskanäle zum Anschließen der Spülleitung oder der Werkzeuge, da diese aufgrund der geringen Durchmesser und komplexen Geometrie nur schwierig zugänglich sind. Zur Dekontamination von Endoskopen stehen im Prinzip drei Verfahrensvarianten zur Verfügung:
a) das Autoklavieren
b) die Ethylenoxidsterilisation und
c) die Plasmasterilisation.

Ein Problem beim Autoklavieren von Endoskopen besteht darin, dass aufgrund der hohen Temperaturen (>130°C) eine Trübung der Optik zur Bildübertragung eintreten kann, die aus einem Kondensieren von während dem Autoklavieren herausgelösten Kohlenstoffmolekülen resultiert.

Die vorgenannte Ethylenoxidsterilisation wird heutzutage praktisch in Krankenhäusern und Arztpraxen nicht mehr angewendet, da zum einen die Sterilisation sehr lange dauert und zum anderen eine mehrstündige Belüftungsphase erforderlich ist, um toxische Rückstände zu eliminieren.

In jüngster Zeit hat sich daher die Plasmasterilisation durchgesetzt, bei der die Mikroorganismen durch Bildung von hochreaktiven Hydroxy- und Hydroxylradikalen aus Wasserstoffperoxid abgetötet werden. Dieser Sterilisationsprozess läuft im Vakuum bei einer vergleichsweise geringen Temperatur (etwa 45°C) ab, so dass keine thermische Schädigung der Optik erfolgt. Nachteil dieses Verfahrens ist jedoch, dass Kanülen oder Sonden mit Lumina unter 0.3 cm und über 30 cm Länge nicht mit einer hinreichenden Zuverlässigkeit sterilisiert werden können, da bei derartigen Lumina mit geringem Durchmesser-/Längeverhältnis nicht sichergestellt ist, dass der Wasserstoffperoxiddampf zur Sterilisation in den gesamten Lumenraum eingebracht werden kann.

Ein weiteres Problem herkömmlicher Endoskope besteht darin, dass diese - wie vorstehend ausgeführt - im Hinblick auf die jeweilige Anwendung optimiert sind. Dabei müssen beispielsweise zur therapeutischen Endoskopie in Abhängigkeit von der Instrumentierung Endoskope mit unterschiedlichen Sonden- und Kanülendurchmessern bereitgestellt werden, so dass erhebliche Investitionskosten anfallen.

Demgegenüber liegt der Erfindung die Aufgabe zugrunde, ein Endoskop zu schaffen, das zum einen auf einfachste Weise dekontaminierbar ist und das zum anderen einen breiten Anwendungsbereich ermöglicht.

Diese Aufgabe wird durch ein modulares Endoskop mit den Merkmalen des Anspruchs 1 gelöst.

Das modulare Endoskop hat eine Optik zur Bildübertragung, die lösbar mit einer Kanüle verbindbar ist. Das modulare Endoskop hat des Weiteren eine Vielzahl von disposablen Kanülen, die in Abhängigkeit von einer diagnostischen oder therapeutischen Anwendung mit unterschiedlichen Durchmessern und Anschlüssen ausgeführt sind. Da sich je nach Anwendungsfall die Länge der Kanüle ändern kann, stets jedoch die gleiche Optik verwendet wird, wird erfindungsgemäß eine Ausgleichseinrichtung verwendet werden, über die die Optik mit Bezug zur Kanülen-/Arbeitsschaftmündung ausgerichtet werden kann. Das heißt, je nach Anwendung und Instrumentierung kann eine geeignete Kanüle an die Optik angesetzt werden. Diese Kanülen werden nach dem Gebrauch vernichtet, so dass lediglich die Optik dekontaminiert werden muss. Die Optik hat keine für die Plasmasterilisation kritischen Innenabmessungen, nur eine glatte äußere Oberfläche, so dass die Dekontamination sehr zuverlässig und mit geringem Aufwand nach einem Plasmasterilisationsverfahren bei geringer Temperatur und ohne thermische Beschädigung durchgeführt werden kann.

Eine mechanische Beschädigung der Optik durch äußere Einflüsse kann durch ein aufgesetztes Schutzrohr nahezu ausgeschlossen werden.

Dabei ist durch geeignete Auslegung der Schutzrohrlänge und des Schutzrohrdurchmessers gewährleistet, dass die Optik bei der Plasmasterilisation dekontaminiert werden kann.

Bei einem Ausführungsbeispiel des modularen Endoskops können die je nach Anwendungsfall ausgewählten Kanülen auswechselbar an disposable Mehrport-Anschlussstücke angesetzt werden, die einen Anschluss für die Optik und weitere Anschlüsse, beispielsweise für eine Spüleinrichtung, ein Werkzeug oder dergleichen, haben. Die Anschlussstücke können als Y-Stück mit einem axialen und einem schräg dazu angestellten Anschluss ausgeführt sein, wobei dann der axiale Anschluss in der Regel der Optikanschluss ist.

Bei Anwendungen, die eine Instrumentierung erfordern, wird ein disposable Anschlussstück vorgesehen, bei dem ein axialer Anschluss und zwei schräg dazu angeordnete seitliche Anschlüsse vorgesehen sind, wobei einer der seitlichen Anschlüsse der Optikanschluss und der axiale Anschluss ein Arbeitsanschluss zum Ansetzen eines Werkzeugs ist.

Zum Ansetzen von Arbeitsschäften mit großem Durchmesser wird es bevorzugt, die Mehrport-Anschlussstücke mit einer zusätzlichen geeigneten Kupplung, beispielsweise einem Luer-Lock zu versehen, über die das modulare Endoskop an den Arbeitsschaft angesetzt werden kann.

Im Übergangsbereich vom ummantelten Teil der Optik zu demjenigen Endabschnitt, der in der Kanüle aufgenommen ist, ein Führungsrohr vorgesehen, entlang dem die Ausgleichseinrichtung verschiebbar ist.

Bei sehr kurzen Kanülen kann die Ausgleichseinrichtung zusätzlich mit einem Distanzstück ausgeführt sein, das zwischen das Anschlussstück und die eigentliche, verstellbare Ausgleichseinrichtung eingesetzt wird. Ein derartiges Distanzstück kann beispielsweise bei Kanülen für die Vitrektomie eingesetzt werden, die relativ kurz und gebogen sind.

Dieses Distanzstück kann auch einstückig mit dem Anschlussstück ausgeführt sein.

Bei einer Variante des Endoskops ist die Kanüle als Biopsiezange ausgeführt, wobei an ein distales Ende einer Kanüle ein Zangenkopf angesetzt ist, der mittels eines in der Kanüle geführten Betätigungsdrahts betätigbar ist.

Die Betätigung des Zangenkopfs erfolgt vorzugsweise über eine lösbar an den Betätigungsdraht ansetzbare Betätigungseinrichtung, so dass die Kanüle beispielsweise an ein Anschlussstück angesetzt werden kann und anschließend die Betätigungseinrichtung mit dem Betätigungsdraht verbunden und am Anschlussstück abgestützt wird.

Bei einer alternativen Variante kann die Kanüle mit einer externen Aufnahme für ein Werkzeug versehen sein.

Diese Aufnahme ist vorzugsweise an das distale Ende der Kanüle angesetzt.

Für therapeutische Behandlungen mittels Laserenergie kann eine Laseroptik in die Kanüle eingesetzt und mittels eines Lasershifters mit Bezug zum distalen Ende der Kanüle justiert werden.

Die bei dem erfindungsgemäßen Endoskop verwendete Optik kann auch als Bildsensor ausgeführt werden, so dass nach Art einer Videokamera Bildsignale übertragen werden können.

Bei einem Ausführungsbeispiel ist es bevorzugt, die Optik so führen, dass sie aus der Kanüle heraus, beispielsweise in einen Kanal hinein bewegt werden kann, so dass die Überwachung während einer Behandlung mittels eines Bohrers oder dergleichen vereinfacht ist.

Das modulare Endoskop wird vorzugsweise auch mit einer Bürste zum Reinigen der Bauelemente, insbesondere kleiner Lumen ausgeführt.

Bei einer Variante ist ein angeschliffener Trokar vorgesehen, der in eine Kanüle des modularen Endoskops eingesetzt werden kann und der nach dem Positionieren der Kanüle herausgezogen und durch eine Optik oder dergleichen ersetzt wird.

Bei einem Ausführungsbeispiel der Erfindung ist es vorgesehen, einer Optik Kanülen mit Durchmessern von 0.5 mm, 0.95 mm, 1.1 mm, 1.6 mm und 2.0 mm oder mehr zuzuordnen, so dass eine Vielzahl von Endoskopie-Anwendungen abgedeckt ist.

Die Optik wird vorzugsweise mit Licht- und Bildleitern ausgeführt, wobei die Bildleiter über einen Okularanschluss mit einem modularen Okular (vgl. deutsche Patentanmeldung Wechselokular DE 102 56 673.9) verbunden sind und die Lichtleiter über einen Lichtanschluss an eine Lichtquelle angeschlossen sind.

Das proximale Ende der Endoskopkanüle kann ebenso mit einem Luer-Lock-Anschluss oder einem Bajonettanschluss versehen werden, so dass die Kanüle an einen Arbeitsschaft angeschlossen werden kann. In diesen Arbeitsschaft wird die Endoskopkanüle eingeschoben und mittels männlichem oder weiblichem Luer-Lock- oder Bajonettverschluss verbunden. Dieser Arbeitsschaft wurde vor dieser Prozedur mittels Trokar zum Beispiel in einem kleinen Gelenk platziert. Vor Einbringen der Endoskopkanüle wird selbstverständlich der Trokar aus dem Arbeitsschaft entfernt.

Bei therapeutischen Anwendungen, beispielsweise bei der Behandlung kleiner Gelenke, kann ein schräg angeschliffener Trokar in die Kanüle eingesetzt werden. Nach dem Positionieren der Kanüle wird der Trokar herausgezogen und die vorgeschriebene Optik eingesetzt.

Bei dem erfindungsgemäßen modularen Endoskop ist es vorgesehen, die Kanülen und die Anschlussstücke steril verpackt zur Verfügung zu stellen, wobei die Dekontamination in diesem Fall vorzugsweise werksseitig durch Gassterilisation erfolgt.

Sonstige vorteilhafte Weiterbildungen der Erfindung sind Gegenstand weiterer Unteransprüche.

Im Folgenden wird ein bevorzugtes Ausführungsbeispiel der Erfindung anhand schematischer Zeichnungen näher erläutert. Es zeigen:
Figur 1 eine dreidimensionale Prinzipdarstellung eines erfindungsgemäßen Endoskops;
Figur 2 eine Einzeldarstellung einer Optik des Endoskops aus Figur 1;
Figur 3 eine diagnostische Kanüle des modularen Endoskops;
Figur 4 eine therapeutische Kanüle des modularen Endoskops aus Figur 1;
Figur 5 eine therapeutische Kanüle mit einem größeren Durchmesser als diejenige in Figur 4;
Figur 6 ein Beispiel eines mit der therapeutischen Kanüle gemäß den Figuren 4 und 5 ausgeführten Endoskops;
Figur 7 ein Ausführungsbeispiel eines diagnostischen Endoskops mit einer Kanüle gemäß Figur 2;
Figuren 8 bis 11 ein Ausführungsbeispiel einer Kanüle mit extern angesetztem Werkzeug und
Figur 12 ein Ausführungsbeispiel eines Endoskops mit einer als Biopsiezange ausgeführten Kanüle.

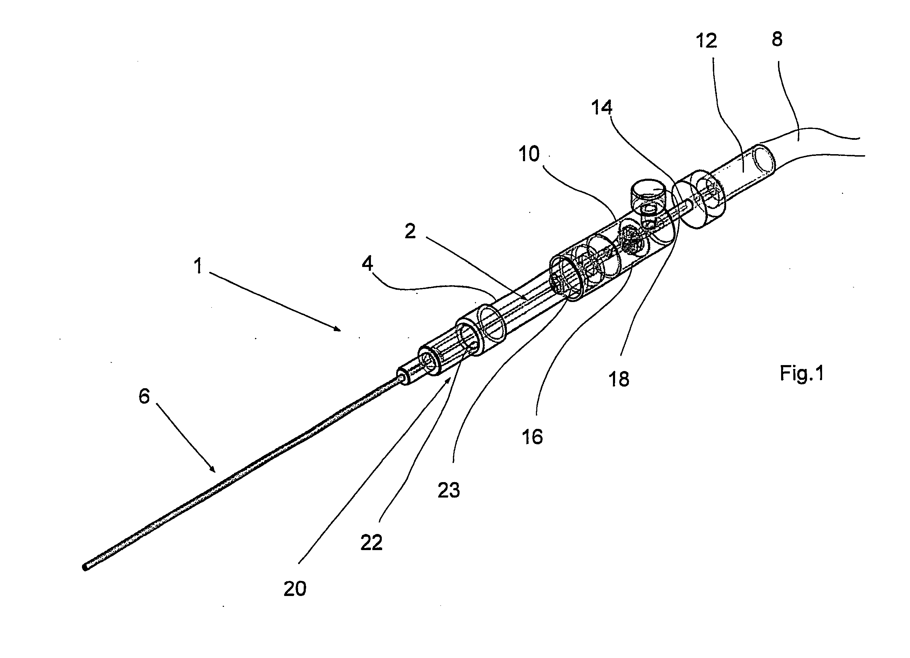

Figur 1 zeigt eine schematisierte dreidimensionale Darstellung eines modularen starren-semiflexiblen Endoskops 1, das in Abhängigkeit vom Verwendungszweck aus den im Folgenden näher beschriebenen Endoskopmoduln zusammengesetzt ist. Ein derartiges Endoskop 1 besteht im Wesentlichen aus einer Optik 2, die über ein Anschlussstück 4 an.eine Kanüle 6 angesetzt ist. Die Optik 2 besteht in an sich bekannter Weise aus einem hochflexiblen Bündel geordneter Bild- und Lichtfasern, über die ein zu untersuchender Bereich beleuchtet und Bildinformationen einem Okular- oder einem angeschlossenen Bildaufzeichnungsgerät zugeleitet werden. Diese Optik 2 kann in Hinblick auf die vorgesehenen Anwendungen mit einer Auflösung zwischen 3.000 und 50.000 Pixeln ausgeführt sein. Der rückwärtige, sich von dem Anschlussstück 2 weg zu einem in Figur 1 nicht dargestellten entkoppelbaren Okular, das beispielsweise auch als Zoomokular ausgeführt sein kann, erstreckende Bereich der Optik 2 ist mit einem Schutzschlauch 8 versehen, der diesen Abschnitt gegen äußere Einflüsse schützt. Im Bereich der Kanüle ist der Schutzschlauch 8 abgelängt, so dass die in einer Hülse aufgenommenen Bild- und Lichtfasern hervorstehen. Der distale dem zu beobachtenden Bereich zugewandte Endabschnitt der Optik 2 ist mit einem Linsensystem versehen und mittels einer Diamantglasscheibe abgedichtet, so dass das Innere der Optik während der Untersuchung nicht kontaminiert werden kann. Hinsichtlich des Aufbaus derartiger Optiken sei beispielsweise auf die eingangs genannte DE 10 2006 022 827 A1 verwiesen.

Die den Endabschnitt der Optik 6 aufnehmende Kanüle 6 ist entweder aus Edelstahl oder einem flexiblen Kunststoff hergestellt, wobei der Anwender zwischen Kanülen im Durchmesserbereich von 0.95 mm bis 2.0 mm oder mehr auswählen kann. Beim beschriebenen Ausführungsbeispiel ist die Optik 2 mit einem Außendurchmesser von etwa 0.5 vorzugsweise 0.53 mm ausgeführt. Dieser sehr geringe Durchmesser ermöglicht den Einsatz des Endoskops 1 beispielsweise im Tränenkanal, im Milchkanal (Krebsfrüherkennung), im Wurzelkanal oder im Speichelkanal.

Die Verbindung zwischen der Optik 8 und der Kanüle 6 erfolgt über das Anschlussstück 4, das bei dem Ausführungsbeispiel gemäß Figur 1 als Ein-Port-Anschluss ausgeführt ist. Bei dem modularen Endoskop 1 ist es vorgesehen, dass einer einzigen Optik 2 einer Vielzahl von unterschiedlichen Kanülen 6 und Anschlussstücken 4 zugeordnet ist. Die Grundabstimmung in Hinblick auf die Länge der Optik und die Länge der Kanüle erfolgt durch geeignetes Ablängen des Schutzschlauchs 8, das auch für den Laien auf einfache Weise durchführbar ist. Der präzise Längenausgleich erfolgt dann über eine Ausgleichseinrichtung, die im Folgenden als Shifter 10 bezeichnet wird. Der Grundaufbau eines derartigen Shifters 10 ist beispielsweise in der DE 10 2005 08 825 B3 beschrieben, so dass hier nur die zum Verständnis wichtigen Elemente beschrieben werden. Der Shifter 10 ist über einen Knickschutzanschluss 12 an die Optik 2, genauer gesagt an den Schutzschlauch 8 angesetzt und hat ein die Optik 2 umgreifendes Führungsrohr 14, entlang dem ein mit der Kanüle 6 verbundenes Gleitstück 16 verschiebbar geführt ist. Dieses lässt sich über eine Feststellschraube 18 auf dem Führungsrohr 14 fixieren, so dass entsprechend die Optik 2 innerhalb der Kanüle 6 verschoben werden kann, bis das distale Ende der Optik 2 mit Bezug zur Kanülenmündung ausgerichtet ist. Die Feststellschraube 18 wird dann angezogen, um diese Relativposition festzulegen. Beim dargestellten Ausführungsbeispiel wird die Kanüle 6 lösbar über eine Kupplung 20 an einen Kanülenanschluss 22 angesetzt. Das Anschlussstück 4 ist über eine geeignete Kupplung 23 lösbar mit dem Shifter 10 verbunden. Für die Kupplungen 22, 23 können beispielsweise Luer-Kupplungen verwendet werden.

Wie bereits erwähnt, können Durchmesser, Länge und Material der Kanüle 6 in Abhängigkeit vom Verwendungszweck aus dem Modul-Bausatz ausgewählt werden. Wie im Folgenden noch näher erläutert wird, sind sowohl die Kanüle 6 als auch das Anschlussstück 4 als Disposable-Bauteil ausgeführt und werden in steriler Verpackung ausgeliefert und nach einmaliger Verwendung entsorgt. Dementsprechend muss lediglich die Optik 2 dekontaminiert werden.

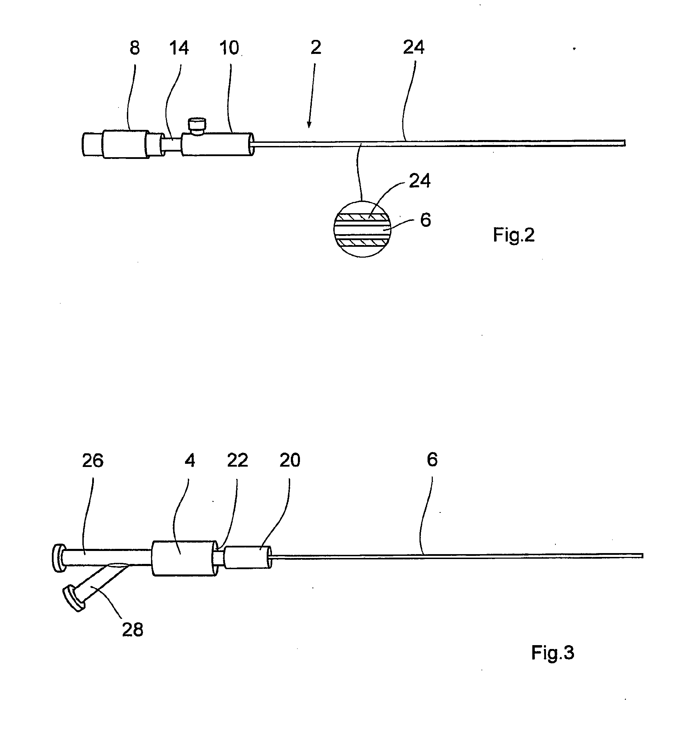

Figur 2 zeigt eine Teildarstellung der Optik 2 aus Figur 1 mit dem Knickschutzanschluss 8, dem Führungsrohr 14, dem Shifter 10 und dem daraus hervorstehenden distalen Endabschnitt der Optik 2, der in Figur 1 in der Kanüle 6 und im Anschlussstück 4 aufgenommen ist. In der Detaildarstellung gemäß Figur 2 ist zum Schutz der empfindlichen Bild- und Lichtleiter sowie der Diamantglasscheibe und der Linsenanordnung ein Schutzrohr 24 aufgeschoben. Dieses überstreckt die gesamte Länge des distalen Endabschnitts der Optik 2, so dass eine Beschädigung ausgeschlossen ist.

Das Schutzrohr 24 ist beim dargestellten Ausführungsbeispiel aus einem metallischen Werkstoff hergestellt und verbleibt während der Lagerung, des Transports und auch der Dekontamination auf dem distalen Endabschnitt der Optik 2. Innendurchmesser und Länge dieses Schutzrohrs 24 sind so gewählt, dass eine Plasmasterilisation durch Wasserstoffperoxid durchgeführt werden kann.

Zum Ansetzen einer Kanüle 6 an die sterilisierte Optik 2 wird zunächst das Schutzrohr 24 abgenommen und der distale Endabschnitt der Optik 2 in eine Kanüle 6 eingeführt. Figur 3 zeigt ein Ausführungsbeispiel für eine diagnostische Kanüle 6, die über eine Luer-Kupplung 20 an den Kanülenanschluss 22 eines Anschlussstücks 10 angesetzt ist, das mit einem axialen Anschluss oder Ausgang 26 und einem Schräganschluss 28 ausgeführt ist. Bei einer derartigen diagnostischen Kanüle 6 wird die Optik 2 vorzugsweise in den axialen Anschluss 26 eingesetzt, während der Anschluss 28 zum Anschluss einer Spülmittelleitung dient.

Der Durchmesser der Kanüle 6 ist dabei an den jeweiligen Anwendungsfall angepasst. Bei dem dargestellten Ausführungsbeispiel ist das Anschlussstück 10 aus Kunststoff gefertigt, während die Kanüle 6 und der kanülenseitige Teil der Kupplung 20 (Luer-Lock) aus Stahl hergestellt sind.

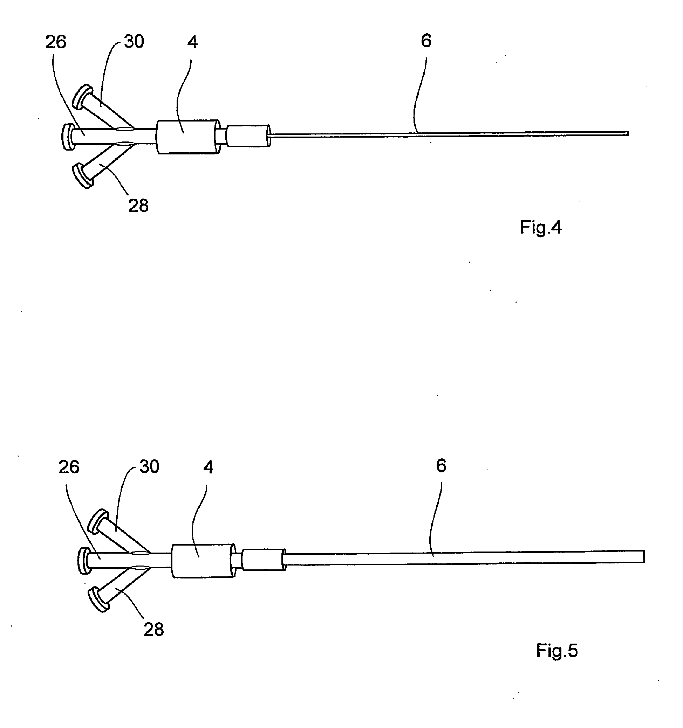

Figur 4 zeigt ein Ausführungsbeispiel einer therapeutischen Kanüle, bei der das Anschlussstück 4 mit drei Anschlüssen: einem axialen Anschluss 26, einem Schräganschluss 28 und einem symmetrisch zu diesem angeordneten weiteren Schräganschluss 30 ausgeführt ist. Bei dieser Variante wird die Optik 2 in einen der Schräganschlüsse 28, 30 eingesetzt. Durch den mittigen axialen Anschluss 26 kann beispielsweise ein Werkzeug, wie eine Biopsie-Zange, ein Körbchen zum Entfernen von Steinen oder ein Mikrobohrer angesetzt werden, wie er in der DE 197 44 856 beschrieben ist. An den verbleibenden Schräganschluss 28, 30 kann dann wiederum eine Spülleitung angeschlossen werden.

Im Übrigen entspricht die Kanüle gemäß Figur 4 derjenigen aus Figur 3, so dass weitere Erläuterungen entbehrlich sind.

Figur 5 zeigt eine Variante der therapeutischen Kanüle gemäß Figur 4, wobei an das Anschlussstück 10 mit den drei Ausgängen oder Anschlüssen 26, 28, 30 eine Kanüle 6 mit einem wesentlich größeren Durchmesser als beim Ausführungsbeispiel gemäß Figur 4 angesetzt ist. Der größere Kanülendurchmesser ermöglicht beispielsweise den Einsatz eines größeren Werkzeugs.

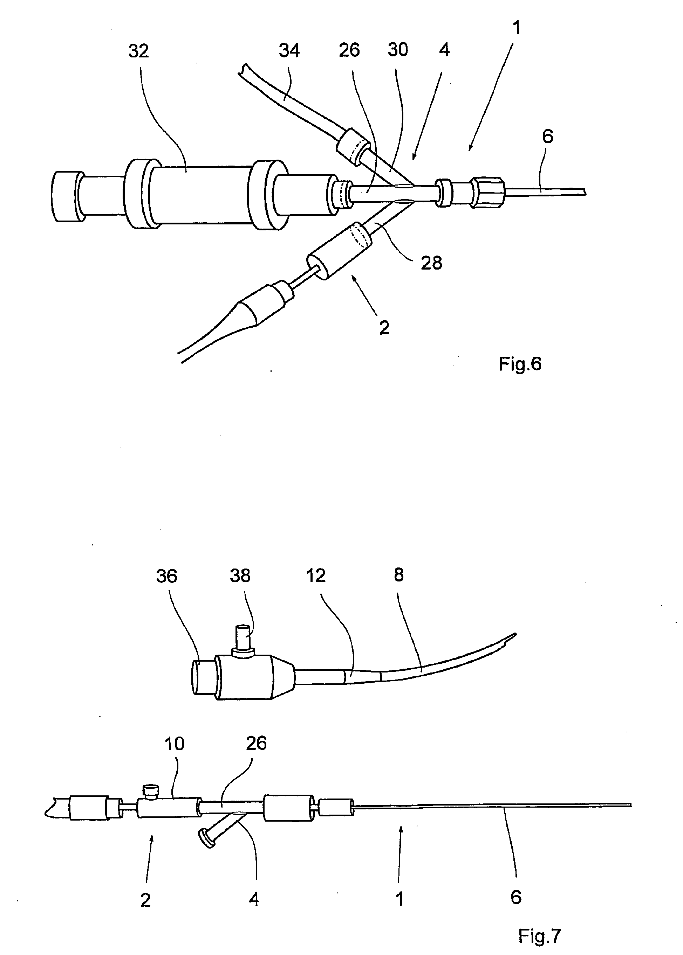

Figur 6 zeigt eine Teildarstellung eines Endoskops 1, das durch Kombination der Optik aus Figur 2, einer Kanüle 6 und einem Anschlussstück 10 gemäß Figur 4 als therapeutische Sonde 1 ausgeführt ist. Man sieht bei diesem Ausführungsbeispiel, dass die Optik 2 an den Schräganschluss 28 angeschlossen ist. Dies ist nur möglich, da bei dem beschriebenen modularen System die Optik 2 so flexibel ausgestaltet ist, dass sie ohne Beschädigung durch den Schräganschluss 28 hindurch in die Kanüle 6 eingeführt werden kann. Hierzu wird das Anschlussstück 4 zumindest im Bereich zwischen dem Schräganschluss 28 und den sich daran anschließenden Axialbereich glattflächig ausgeführt, so dass die Optik 2 gleitend eingesetzt werden kann. An den mittigen axialen Ausgang 26 ist bei diesem Ausführungsbeispiel ein Bohrwerkzeug 32 angesetzt. An den weiteren Schräganschluss 30 ist eine Spülleitung 34 angeschlossen.

Figur 7 zeigt eine Teildarstellung eines diagnostischen Endoskops 1 mit einer Kanüle 6 und einem Anschlussstück 4 gemäß Figur 3, wobei in den axialen Ausgang 26 die Optik 2 eingesetzt ist und mittels des Shifters 10 mit Bezug zur Mündung der Kanüle 6 ausgerichtet ist. Der sich an den Shifter 10 nach hinten, weg von der Kanüle 6 anschließende Bereich der Optik 2 ist - wie vorstehend erläutert - mit dem Schutzschlauch 8 ummantelt. Der von der Kanüle 6 entfernte Endabschnitt der Optik 2 ist über einen weiteren Knickschutzanschluss 12 an eine Okularkupplung 36 angeschlossen ist, an der auch ein Beleuchtungsanschluss 38 für die Lichtleiter der Optik 2 vorgesehen ist. Über die Okularkupplung 36 kann die Optik 2 an ein Okular, beispielsweise ein Zoom-Okular angesetzt werden. Dieses Zoom-Okular ermöglicht es, trotz des geringen Außendurchmessers von 0.53 mm der Optik 2, das Bild mit einem hinreichenden Durchmesser von mehr als 11 cm abzubilden, wobei durch geeignete Filter Moire-Effekte unterdrückt werden können.

Prinzipiell ist es bei dem erfindungsgemäßen modularen Endoskopsystem vorgesehen, die Kanülen 6 und die Anschlussstücke 4 jeweils nach jedem Gebrauch wegzuwerfen, da insbesondere bei Kanülen mit geringem Durchmesser aufgrund des sehr kleinen Lumendurchmessers eine Dekontamination durch eine Plasmabehandlung nicht möglich ist. Für besondere Anwendungen sieht es das modulare System vor, auch Kanülen 6 und Anschlussstücke 4 der vorbeschriebenen Bauart zur Verfügung zu stellen, die wiederverwertbar sind und die aus einem autoklavierbaren Material hergestellt sind.

Beim beschriebenen Ausführungsbeispiel ist es auch vorgesehen, sowohl an therapeutischen als auch an diagnostischen Anschlussstücken 4 kanülenseitig einen weiteren, axialen Luer-Lock vorzusehen. Dieser hat einen größeren Durchmesser als die Kupplung 20 (siehe Figur 3), über die die Kanüle 6 ans Anschlussstück 4 angesetzt wird. Dieser zusätzliche Luer-Lock ermöglicht es, das Endoskop 1 an einen Arbeitsschaft mit vergleichsweise sehr großem Durchmesser anzusetzen. So wird beispielsweise bei der Arthroskopie ein derartiger Arbeitsschaft mit vergleichsweise großem Durchmesser gemeinsam mit einem Trokar in das Gelenk eingeführt, um eine Öffnung zum Einführen des Endoskops auszubilden. Nach dem Positionieren des Arbeitsschafts im Gelenk wird der Trokar herausgezogen - der Arbeitsschaft verbleibt im Gelenk. Anschließend wird das Endoskop 1 angesetzt und über den zusätzlichen Luer-Lock mit dem Arbeitsschaft verbunden.

Bei der Operation an kleineren Gelenken kann ein vergleichsweise kleiner, angeschliffener Trokar in die Kanüle 6 eingesetzt werden. Nach dem Positionieren der Kanüle 6 mittels des Trokars in dem Gelenk wird der Trokar aus der Kanüle 6 herausgezogen und die vorbeschriebene Optik 2 sowie das Operationswerkzeug, beispielsweise ein Bohrer oder ein Faserstrang zur Übertragung von Laserlicht oder dergleichen über das Anschlussstück 4 in die Kanüle 6 eingeführt.

Bei bestimmten Therapien, beispielsweise bei der Vitrektomie werden Kanülen eingesetzt, die im Vergleich zur "Standard"-Optik 2 wesentlich kürzer sind, so dass der Shifter 10 nicht ausreicht, um einen Längenausgleich herbeizuführen. In diesem Fall kann zwischen das Anschlussstück 4 und den Shifter 10 ein Distanzstück (nicht dargestellt) eingesetzt werden, dessen Länge so gewählt ist, dass die resultierende Länge aus der beispielsweise gebogenen Kanüle und dem Distanzstück etwa der Länge einer vorbeschriebenen Kanüle 6 entspricht - die Position der Optik 2 kann dann, wie vorbeschrieben, über den Shifter 10 eingestellt werden.

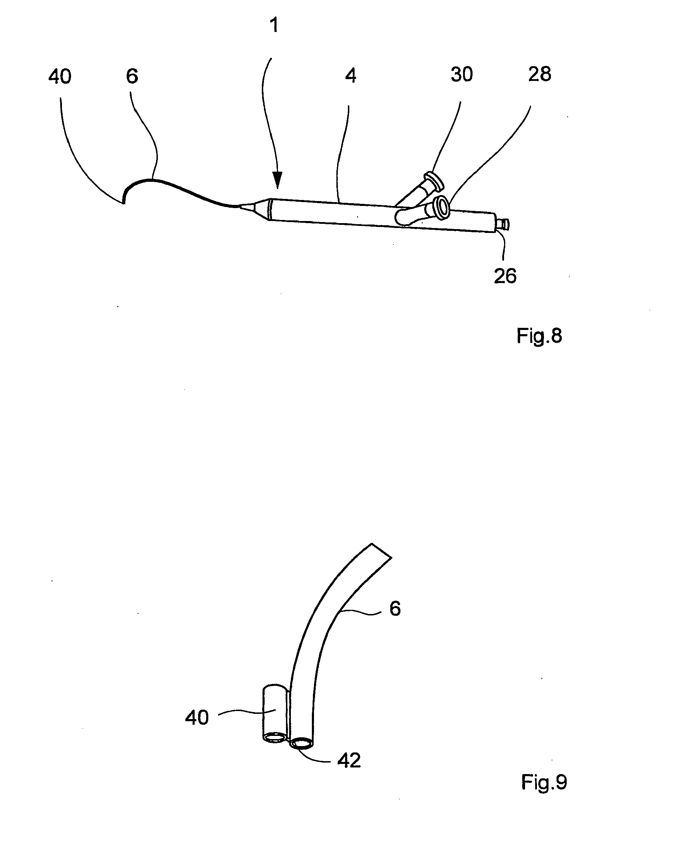

Anhand der Figuren 8 bis 11 wird ein Ausführungsbeispiel des modularen Endoskops 1 mit gebogener Kanüle 6 erläutert, wie es beispielsweise bei der Zahnwurzelbehandlung eingesetzt werden kann. Bei einer derartigen Zahnwurzelbehandlung wird ein Bohrwerkzeug mittels der vorbeschriebenen Optik 2 im Wurzelkanal positioniert. Prinzipiell kann man für eine derartige Behandlung eine gebogene therapeutische Kanüle einsetzen, deren Aufbau gemäß den vorbeschriebenen Ausführungsbeispielen ausgeführt ist. Dabei kann dann beispielsweise wie beim Ausführungsbeispiel gemäß Figur 4 ein Mikrobohrer an einen axialen Anschluss 26 angesetzt werden, während an die beiden Schräganschlüsse 28, 30 die Optik und eine Spülleitung angeschlossen werden. Figur 8 zeigt jedoch ein Ausführungsbeispiel, bei dem das Werkzeug außen an der Kanüle 6 festgelegt ist.

Bei diesem Endoskop ist eine gebogene kurze Kanüle 6 an ein Anschlussstück 4 mit einem axialen Anschluss 26 und zwei V-förmig zueinander angeordneten, in der Darstellung gemäß Figur 8 nach oben versetzten Schräganschlüssen 28, 30 angesetzt. Dieses Anschlussstück 4 hat eine größere Axiallänge als diejenigen der vorbeschriebenen Ausführungsbeispiele, so dass die geringe Axiallänge der gebogenen Kanüle 6 ausgeglichen ist. An dem distalen Endabschnitt der gebogenen Kanüle 6 ist eine Aufnahme 40 für das externe Ansetzen eines Werkzeugs, beispielsweise eines Nickeltitanbohrers, wie er bei Wurzelkanalbehandlungen üblicherweise eingesetzt wird, vorgesehen.

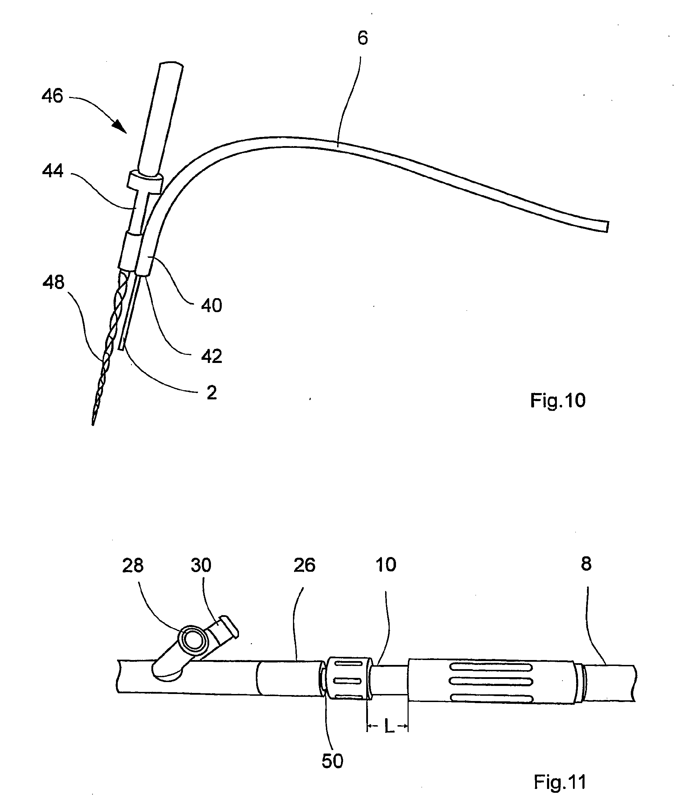

Dieser distale Endabschnitt der Kanüle 6 ist in Figur 9 vergrößert dargestellt. Demgemäß ist im Mündungsbereich 42 der Kanüle 6 die bei diesem Ausführungsbeispiel etwa hülsenförmige Aufnahme 40, beispielsweise durch Löten oder dergleichen, angesetzt, wobei die Hülsenachse und die Achse des Kanülen-Mündungsbereiches 42 etwa im Parallelabstand oder etwas angestellt zueinander verlaufen. Wie in Figur 10 dargestellt, kann in diese Aufnahme ein feststehender Schaft 44 eines Bohrwerkzeugs 46 angeordnet werden, das pneumatisch, hydraulisch oder elektrisch angetrieben ist. Ein Bohrer 48 des Bohrwerkzeugs 46 kann dann mit Hilfe der über den axialen Anschluss 26 in die Kanüle 6 eingesetzten Optik 2 im Wurzelkanal positioniert werden.

Gemäß Figur 11 erfolgt die Justierung der Optik 2 mit Bezug zum Mündungsbereich 42 der Kanüle 6 - ähnlich wie bei den zuvor beschriebenen Ausführungsbeispielen - über einen Shifter 10, der an den axialen Anschluss 26 angesetzt ist. Über eine Verstellschraube 15 kann die in Figur 11 angedeutete Justierlänge L eingestellt werden, um die Optik 2 mit Bezug zum Mündungs-bereich 42 zu positionieren. Gemäß Figur 10 muss dabei die Optik 2 nicht mit dem Mündungsbereich 42 abschließen, sondern kann beispielsweise durch Verstellen des Shifters 10 während der Wurzelbehandlung in den Wurzelkanal geschoben werden, so dass die exakte Wirkposition des Bohrers 48 stets beobachtet werden kann. Das heißt, das erfindungsgemäße Endoskop ermöglicht eine variable Positionierung der Optik 2 während der Behandlung.

Selbstverständlich ist diese variable Positionierung auch dann möglich, wenn das Bohrwerkzeug durch die Kanüle 6 hindurch geführt ist.

Bei den zuvor beschriebenen Ausführungsbeispielen wird vorzugsweise eine Glasfaseroptik eingesetzt. Prinzipiell können jedoch auch Bildsensoren verwendet werden, wie sie beispielsweise in der Videotechnik eingesetzt werden (CCD-, CMOS-Sensoren oder dergleichen).

Wie eingangs erläutert, werden beispielsweise bei der Vitrektomie gebogene Kanülen 6 eingesetzt, wobei als Werkzeug dann ein Diodenlaser verwendet wird und der Laserstrahl über Glasfasern zum zu behandelnden Bereich geführt ist. Zur Positionierung dieses Glasfaserstranges wird dann beispielsweise ein Lasershifter verwendet, wie er in der DE 199 56 516 beschrieben ist.

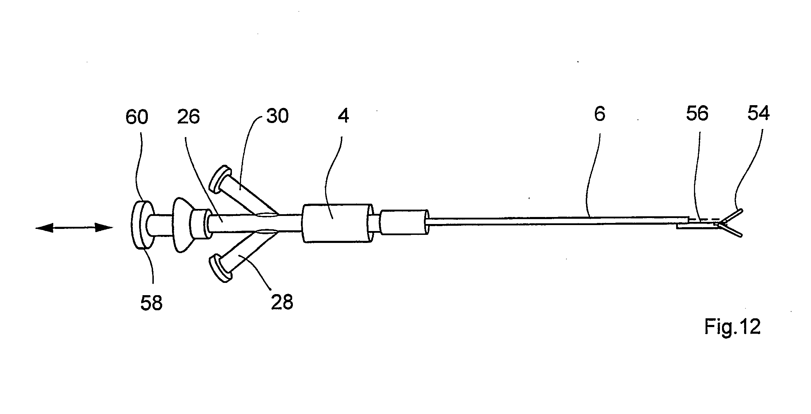

Figur 12 zeigt ein Ausführungsbeispiel, bei dem als Werkzeug eine Biopsiezange verwendet wird. Herkömmliche Biopsiezangen, wie sie beispielsweise in der DE 20 2006 008 061 U1 beschrieben sind, haben einen vergleichsweise großen Durchmesser von mindestens 0,8 Millimetern, so dass es praktisch unmöglich ist, diese Biopsiezange gemeinsam mit einer Optik 2 in eine Kanüle 6 des modularen Endoskops 1 gemäß den vorbeschriebenen Ausführungsbeispielen einzusetzen, da dann der Gesamtdurchmesser der Kanüle 6 für eine Vielzahl von Behandlungen wesentlich zu groß wäre.

Dieser Nachteil wird beim erfindungsgemäßen modularen Endoskop 1 dadurch überwunden, dass eine Kanüle 6 mit einer Biopsiezange ausgeführt wird. Eine derartige Biopsie-Kanüle ist in Figur 12 dargestellt. Dabei ist an eine herkömmliche, gerade Kanüle 6 mit einem Durchmesser von beispielsweise 1,1 Millimetern (oder weniger) ein Biopsie-Zangenkopf 52, beispielsweise durch Löten oder dergleichen angesetzt. Bei dem dargestellten Ausführungsbeispiel ist dieser Zangenkopf 52 seitlich am distalen Ende der Kanüle 6 befestigt. Die Betätigung von Zangenbacken 54 (beispielsweise Löffeln) zur Entnahme einer Gewebeprobe, erfolgt über einen in Figur 12 gestrichelt angedeuteten Betätigungsdraht 56, der sich durch die Kanüle 6 und das Anschlussstück 4 sowie den axialen Anschluss 26 hindurch zu einer Betätigungseinrichtung 58 erstreckt. In der in Figur 12 dargestellten Position ist ein federvorgespanntes Betätigungselement 60 gegen die Federkraft nach rechts bewegt, so dass sich die Zangenbacken 54 V-förmig öffnen. Bei Entlasten des Betätigungselements 60 schließen sich die Zangenbacken 54, so dass die Gewebeprobe entnommen werden kann. Das heißt, bei diesem erfindungsgemäßen Ausführungsbeispiel wird die Funktion eines bei herkömmlichen Biopsiezangen verwendeten spiralförmigen Metallstützschlauchs durch die Kanüle 6 übernommen, so dass die Biopsiezange ohne weiteres an ein Anschlussstück 4 des modularen Endoskops 1 ansetzbar ist. Zum Ansetzen dieser Biopsie-Kanüle an das Anschlussstück 4 kann die Betätigungseinrichtung 58 von dem Betätigungsdraht 56 gelöst werden, so dass dessen von den Zangenbacken 54 entfernter Endabschnitt beim Ansetzen der Kanüle 6 an das Anschlussstück 4 durch den axialen Anschluss 26 hindurchgeführt werden kann und anschließend die Betätigungseinrichtung 58, beispielsweise durch Schrauben oder Klemmen, mit dem Betätigungsdraht 56 verbunden wird. Die Optik 2 kann dann wieder durch einen der seitlichen Anschlüsse 28, 30 eingesetzt werden.

Die vorbeschriebene Biopsie-Kanüle ist vorzugsweise aus Metall gefertigt und kann ohne größere Probleme autoklaviert oder auf sonstige Weise sterilisiert werden.

In dem Fall, in dem Elemente des modularen Endoskops dekontaminiert werden sollen, gehören zu diesem System an die jeweilige Geometrie angepasste Reinigungsbürsten, um auch schlecht zugängliche Bereiche bspw. kleine Lumen zu reinigen.

Das erfindungsgemäße vorbeschriebene modulare Konzept ermöglicht mit einer einzigen Optik und unterschiedlichen disposable Kanülen/Arbeitsschäften nahezu jede Instrumentierung. Dabei können unterschiedliche Arbeitslängen des Endoskops auf einfache Weise durch Verlängern oder Verkürzen des außen liegenden Schutzschlauchs realisiert werden. Den Anwendern wird es somit ermöglicht, interdisziplinär zu arbeiten und auch Arbeitsinstrumente aus anderen Fachbereichen durch einfache Anpassung des Endoskops einzusetzen. Durch die Ein-Mal-Verwendung von Kanülen 6 und Anschlussstücken 4 und die einfache Sterilisation der Optik 2 ist eine Dekontamination des Endoskops in vorbildlicher Weise gewährleistet, wobei der Aufwand für die Anwender minimal ist, so dass auch die Investitionskosten gegenüber herkömmlichen Lösungen erheblich geringer sind.

Offenbart ist ein modulares Endoskop-System, bei dem einer einzigen Optik eine Vielzahl von Anschlussstücken, Werkzeugen und Kanülen zugeordnet sind, die je nach Verwendung ausgewählt werden. Die Anschlussstücke und die Kanülen sind dabei vorzugsweise für eine Ein-Mal-Anwendung ausgelegt, so dass lediglich die Optik dekontaminiert werden muss.

Die Anmelderin behält sich vor, auf die einzelnen Ausführungsbeispiele der beschriebenen Endoskopmodule jeweils eigene, unabhängige Patentansprüche zu richten. Das heißt, jedes der in den Figuren dargestellten Ausführungsbeispiele kann zum Gegenstand einer eigenen, unabhängigen Patentanmeldung gemacht werden.

### Bezugszeichenliste

- 1: Endoskop
- 2: Optik
- 4: Anschlussstück
- 6: Kanüle
- 8: Schutzschlauch
- 10: Shifter
- 12: Knickschutzanschluss
- 14: Führungsrohr
- 16: Gleitstück
- 18: Feststellschraube
- 20: Kupplung
- 22: Kanülenanschluss
- 23: Kupplung
- 24: Schutzrohr
- 26: axialer Anschluss
- 28: Schräganschluss
- 30: weiterer Schräganschluss
- 32: Bohrwerkzeug
- 34: Spülleitung
- 36: Okularanschluss
- 38: Beleuchtungsanschluss
- 40: Aufnahme
- 42: Mündungsbereich
- 44: Schaft
- 46: Bohrwerkzeug
- 48: Bohrer
- 50: Verstellschraube
- 52: Zangenkopf
- 54: Zangenbacken
- 56: Betätigungsdraht
- 58: Betätigungseinrichtung
- 60: Betätigungselement

## Patentansprüche

1. Modulares Endoskop mit zumindest einer Kanüle (6), durch deren Innenraum eine Optik (2) zur Bildübertragung geführt ist, wobei die Optik (2) mittels einer Kupplung lösbar mit der Kanüle (6) verbunden ist, und mit einer Vielzahl von disposable, sterilisierten Kanülen (6) oder Arbeitsschäften, die in Abhängigkeit von einer diagnostischen oder therapeutischen Anwendung mit unterschiedlichen Durchmessern und/oder Anschlüssen ausgeführt sind, **dadurch gekennzeichnet, dass** den Kanülen (6) gemeinsam eine Optik (2) zugeordnet ist, und dass eine Ausgleichseinrichtung (10) zum Positionieren der linsenseitigen Endabschnitte der Optik (2) mit Bezug zur Kanülen-/Arbeitsschaftmündung vorhanden ist, wobei die Ausgleichseinrichtung (10) einerseits mit der Kanüle (6) oder einem Anschlussstück (4) und andererseits mit der Optik (2) verbunden ist.

2. Endoskop nach Patentanspruch 1, mit einem Schutzrohr (24) zum Aufsetzen auf einen nicht ummantelten Endabschnitt der Optik (2).

3. Endoskop nach einem der vorhergehenden Patentansprüche, mit einem disposable Mehrport-Anschlussstück (4), an das die Kanüle (6) auswechselbar ansetzbar ist und das eine Vielzahl von Anschlüssen (26, 28, 30) für die Optik (2), eine Spülleitung (34), ein Werkzeug (32) oder dergleichen hat.

4. Endoskop nach Patentanspruch 3, wobei das Anschlussstück (4) einen axialen und zwei Y-förmig schräg dazu angeordnete Anschlüsse (26, 28, 30) hat, wobei der axiale Anschluss (26) ein Arbeitsanschluss und einer der Schräganschlüsse (26) ein Optikanschluss ist.

5. Endoskop nach Patentanspruch 3 oder 4, wobei am axialen Anschluss (26) eine zusätzliche Kupplung zum Ansetzen einer Kanüle (6) oder eines Arbeitsschaftes mit großem Durchmesser vorgesehen ist.

6. Endoskop nach einem der vorhergehenden Patentansprüche, wobei die Kanüle (6) mit einer Aufnahme (40) für ein Werkzeug (46) versehen ist.

7. Endoskop nach einem der vorhergehenden Patentansprüche, mit einer Laseroptik, deren Faserstrang in die Kanüle (6) eingesetzt ist und deren Relativposition mit Bezug zu einem distalen Ende der Kanüle (6) mittels eines Lasershifters verstellbar ist.

8. Endoskop nach einem der vorhergehenden Patentansprüche, wobei die Optik mit einem Bildsensor ausgeführt ist.

9. Endoskop nach einem der vorhergehenden Ansprüche rückbezogenen Ansprüche, wobei die Optik aus der Kanüle (6) heraus verschiebbar und festlegbar ist.

10. Endoskop nach einem der vorhergehenden Patentansprüche, wobei die Optik (2) einen Außendurchmesser von weniger als 1 mm, vorzugsweise von etwa 0.5 mm hat und Kanülen (6) mit Durchmessern von 0.95, 1.1, 1.6 und 2.0 mm und mehr vorgesehen sind.

11. Endoskop nach einem der vorhergehenden Patentansprüche, wobei die Kanüle (6) an ihrem proximalen Ende einen Anschluss, insbesondere einen Bajonett- oder Luer-Lock-Anschluss aufweist.

12. Endoskop nach Patentanspruch 11, wobei an dem Kanülenanschluss ein Arbeitsschaft anbringbar ist, der insbesondere dazu ausgelegt ist, einen Trokar aufzunehmen.

13. Endoskop nach einem der vorhergehenden Patentansprüche, wobei zumindest die Kanüle (6) und ein Anschlussstück (4) steril verpackt sind.

## Claims

1. A modular endoscope comprising at least one cannula (6) with optics (2) guided through the interior thereof for image transmission, wherein the optics (2) is detachably connected with the cannula (6) by means of a coupling, having a plurality of disposable, sterilized cannulas (6) or working shafts which are designed with different diameters and/or connections depending on a diagnostic or therapeutic application, **characterized in that** the cannulas (6) have one optics (2) assigned jointly, and that an adjustment means (10) for positioning the lens-side end portions of said optics (2) with respect to the cannula / working shaft orifice is present, wherein said adjustment means (10) is, on the one hand, connected with said cannula (6) or a connecting piece (4) and, on the other hand, with said optics (2).

2. The endoscope according to claim 1, comprising a protective pipe (24) for placing on an uncoated end portion of said optics (2).

3. The endoscope according to any one of the preceding claims, comprising a disposable multi port connecting piece (4) to which said cannula (6) is adapted to be attached exchangeably, and which comprises a plurality of connections (26, 28, 30) for said optics (2), a rinsing pipe (34), a tool (32), or the like.

4. The endoscope according to claim 3, wherein said connecting piece (4) comprises an axial connection and two connections inclined thereto in a Y-shaped manner (26, 28, 30), wherein said axial connection (26) is a working connection and one of said inclined connections (26) is an optics connection.

5. The endoscope according to claims 3 or 4, wherein an additional coupling for attaching a cannula (6) or a working shaft with a large diameter is provided at said axial connection (26).

6. The endoscope according to any one of the preceding claims, wherein said cannula (6) is provided with a receiver element (40) for a tool (46).

7. The endoscope according to any one of the preceding claims, comprising laser optics, the fiber strand of which is inserted in said cannula (6) and the relative position of which is adapted to be adjusted with respect to a distal end of said cannula (6) by means of a laser shifter.

8. The endoscope according to any one of the preceding claims, wherein said optics is designed with an image sensor.

9. The endoscope according to any one of the preceding claims, wherein said optics is adapted to be shifted out of said cannula (6) and to be fixed.

10. The endoscope according to any one of the preceding claims, wherein said optics (2) has an outer diameter of less than 1 mm, preferably of approximately 0.5 mm, and wherein cannulas (6) with diameters of 0.95, 1.1, 1.6, and 2.0 mm and more are provided.

11. The endoscope according to any one of the preceding claims, wherein said cannula (6) comprises a connection, in particular a bayonet or Luer Lock connection, at the proximal end thereof.

12. The endoscope according to claim 11, wherein a working shaft is adapted to be applied at said cannula connection, said working shaft being in particular designed to accommodate a trocar.

13. The endoscope according to any one of the preceding claims, wherein at least said cannula (6) and a connecting piece (4) are packed in a sterile manner.

## Revendications

1. Endoscope modulaire comportant au moins une canule (6) dans l'espace intérieur de laquelle un système optique (2) pour la transmission d'images est conduit, le système optique (2) étant relié de façon amovible à la canule (6) au moyen d'un couplage et comportant une multiplicité de canules jetables stérilisées (6) ou des tiges de travail qui sont conçues en fonction d'une utilisation diagnostique ou thérapeutique avec différents diamètres et/ou raccords, **caractérisé en ce qu'**un système optique (2) est affecté aux canules (6) conjointement et **en ce qu'**un système de compensation (10) pour le positionnement des parties terminales relatives aux lentilles du système optique (2) est présent en lien avec une ouverture de canule/ de tige de travail, le système de compensation (10) étant relié d'une part à la canule (6) ou à une pièce de raccordement (4) et d'autre part au système optique (2).

2. Endoscope selon la revendication 1, comportant un tube protecteur (24) pour le placement sur une partie terminale non gainée du système optique (2).

3. Endoscope selon l'une des revendications précédentes, comportant une pièce de raccordement jetable à plusieurs orifices (4) à laquelle la canule (6) peut être montée de façon échangeable et qui a une multiplicité de raccords (26, 28, 30) pour le système optique (2), une conduite de rinçage (34), un outil (32) ou similaire.

4. Endoscope selon la revendication 3, dans lequel la pièce de raccordement (4) a un raccord axial et deux raccords en forme de Y disposés perpendiculairement à celui-ci (26, 28, 30), le raccord axial (26) étant un raccord de travail et l'un des raccords perpendiculaires (26) étant un raccord optique.

5. Endoscope selon la revendication 3 ou 4, dans lequel sur le raccord axial (26) est prévu un couplage supplémentaire pour fixer une canule (6) ou une tige de travail à gros diamètre.

6. Endoscope selon l'une des revendications précédentes, dans lequel la canule (6) est prévue avec un logement (40) pour un outil (46).

7. Endoscope selon l'une des revendications précédentes, comportant une optique laser dont un câble de fibres est utilisé dans la canule (6) et dont la position relative par rapport à une extrémité distale de la canule (6) peut être déplacée par le biais d'un moyen de décalage de laser.

8. Endoscope selon l'une des revendications précédentes, dans lequel le système optique est conçu avec un capteur d'images.

9. Endoscope selon l'une des revendications précédentes, dans lequel le système optique peut être sorti de la canule (6) et peut être fixé.

10. Endoscope selon l'une des revendications précédentes, dans lequel le système optique (2) a un diamètre externe inférieur à 1 mm, de préférence, d'environ 0,5 mm et les canules (6) sont prévues avec un diamètre de 0,95, 1,1, 1,6 et 2,0 mm et plus.

11. Endoscope selon l'une des revendications précédentes, dans lequel la canule (6) présente à son extrémité proximale, un raccord, en particulier, un raccord à baïonnette ou un raccord Luer.

12. Endoscope selon la revendication 11, dans lequel sur le raccord de canule, une tige de travail peut être montée, qui est conçue en particulier pour recevoir un trocart.

13. Endoscope selon l'une des revendications précédentes, dans lequel au moins la canule (6) et une pièce de raccordement (4) sont conditionnées de façon stérile.
